(19) **Europäisches Patentamt**

**European Patent Office**

**Office européen des brevets**

(11) **EP 1 241 134 A2**

(12) **EUROPÄISCHE PATENTANMELDUNG**

| | |
|---|---|
| (43) Veröffentlichungstag:<br>**18.09.2002 Patentblatt 2002/38** | (51) Int Cl.[7]: **C01B 33/18**, C01B 33/193,<br>B01J 21/08, A61K 9/14 |

(21) Anmeldenummer: **02001655.6**

(22) Anmeldetag: **24.01.2002**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **16.03.2001 DE 10112651**

(71) Anmelder: **Degussa AG**
**40474 Düsseldorf (DE)**

(72) Erfinder:
• **Uhrlandt, Stefan, Dr.**
**53859 Niederkassel (DE)**
• **Schmoll, Ralf, Dr.**
**53125 Bonn (DE)**
• **Drexel, Claus-Peter**
**63263 Neu-Isenburg (DE)**

(54) **Inhomogene Kieselsäuren als Trägermaterial**

(57)     Die Erfindung betrifft Kieselsäuren mit inhomogener Struktur bzw. Aufbau, Verfahren zu deren Herstellung und deren Verwendung als Trägermaterial für Vitamine oder Cholinchlorid.

**EP 1 241 134 A2**

**Beschreibung**

Die Erfindung betrifft Kieselsäuren mit inhomogener Struktur bzw. Aufbau, Verfahren zu deren Herstellung und deren Verwendung als Trägermaterial.

Leicht dispergierbare Kieselsäuren werden z. B. gemäß EP 0 901 986 oder EP 0 647 591 durch Fällung von Wasserglas mit Schwefelsäure und anschließender Trocknung hergestellt. Die getrockneten Produkte werden anschliessend vermahlen und/oder granuliert.

Durch eine mechanische Granulation kann jede Kieselsäure staubfrei hergestellt werden, jedoch verschlechtert sich in der Regel durch diesen zusätzlichen Verfahrensschritt die Dispergierbarkeit.

In einem anderen Verfahren werden Kieselsäuren ebenfalls durch saure Fällung hergestellt, jedoch mittels Verdüsung in Heißluft getrocknet und gleichzeitig zu leicht zerstörbaren Kugeln geformt. So beschreibt EP 018 866 die Herstellung von sprühgetrockneter Kieselsäure mit einem mittleren Partikeldurchmesser von über 80 $\mu$m, wobei die Partikel eine homogene Struktur besitzen und massiv sind.

Sprühgetrocknete Kieselsäuren gemäß EP 0 018 866 eignen sich besonders als Trägermaterial, da sie staubfrei sind und ein hohes Saugvermögen besitzen. Die Staubfreiheit ist für die Verarbeitung der Kieselsäure ein wichtiges Kriterium, da eine einfache Verarbeitung der Kieselsäuren ohne entsprechende Absauganlagen eine hohe wirtschaftliche Bedeutung hat. Neben der Staubfreiheit sind die spezifischen Oberflächen (BET, CTAB) und die Ölaufnahmekapazität (DBP) für die Verwendung als Trägermaterial wichtig.

Im Gegensatz zur mechanischen Granulation können nicht alle Kieselsäuren durch Sprühtrocknung staubfrei hergestellt werden.

Nun erfüllt ein Kieselsäuretyp in der Regel nicht alle geforderten Kriterien. Eine Mischung aus mehreren Kieselsäuretypen ist häufig nur mit einem zu großen Staubanteil herzustellen.

Es wäre wünschenswert, eine Kieselsäure herzustellen, die gleichzeitig breite Bereiche von hysikalisch-chemischen Daten wie BET-, oder CTAB-Oberfläche abdeckt, eine gute Saugfähigkeit und einen geringen Staubanteil aufweist. Wie bereits ausgeführt, kann dies nicht für alle Kieselsäuren durch Sprühtrocknung oder Granulation erreicht werden.

Überraschenderweise wurde gefunden, dass Kieselsäure, die eine inhomogene Zusammensetzung aufweist, leicht auf die geforderten Anforderungen eingestellt werden kann und dennoch eine gute Saugfähigkeit und einen niedrigen Feinanteil aufweist.

Gegenstand der vorliegenden Erfindung sind daher Kieselsäuren, enthaltend mindestens zwei Kieselsäurefraktionen, die sich in mindestens einem Meßwert für BET-Oberfläche, CTAB-Oberfläche und DBP-Aufnahme um mindestens 10 % unterscheiden.

Die erfindungsgemäßen Kieselsäuren sind daher besonders als Trägermaterial für Wirkstoffe wie z. B. Vitamine und Cholinchlorid geeignet.

Der Aufbau der Kieselsäuren aus mindestens zwei Kieselsäurefraktionen bewirkt eine Inhomogenität der Kieselsäure, die sich gleichzeitig in einer guten Saugfähigkeit und einem geringen Feinanteil niederschlägt und die geforderten physikalisch-chemischen Daten ergibt.

Erfindungsgemäße Kieselsäuren besitzen einen Feinanteil von höchstens 10 % mit Teilchendurchmesser kleiner oder gleich 63 $\mu$m (Alpine Siebrückstand).

Ein ähnliches Konzept wird in EP 0 942 029 verfolgt. Hier werden Kompositionen beschrieben, die eine Fällungskieselsäure in zwei verschiedenen Aggregatgrößen enthalten. Die verschiedenen Aggregatgrößen werden für die leichte Dispergierbarkeit der Kieselsäure in einer Gummimischung eingesetzt. Die unterschiedlichen Kieselsäurefraktionen der vorliegenden Erfindung sind nicht in dieser Publikation beschrieben; außerdem ist eine unterschiedliche Aggregatgröße der Kieselsäurefraktionen im vorliegenden Fall von nebengeordneter Bedeutung, wichtig sind die Unterschiede der physikalisch-chemischen Daten. Die Verwendung von Kieselsäuren als Trägermaterial ist in EP 0 942 029 nicht beschrieben. "Kieselsäurefraktion" im Sinne der vorliegenden Erfindung bezeichnet verschiedene Sorten von Kieselsäuren, die aufgrund verschiedener Herstellungsverfahren oder -Varianten einen Unterschied von 10 % in mindestens einer der o. g. physikalisch-chemischen Daten aufweisen. Bevorzugt weisen zwei, besonders bevorzugt drei dieser Parameter einen solchen Unterschied auf.

Die Unterschiede in den bereits genannten Parametern können durch unterschiedliche Herstellverfahren der Kieselsäurefraktionen erhalten werden. So können alle, ein oder mehrere der Kieselsäurefraktionen Fällungskieselsäuren und/oder pyrogene Kieselsäuren sein. Bei Fällungskieselsäuren ist es vor allem möglich, durch verschiedene Fällungsverfahren verschiedene Kieselsäurefraktionen zu erhalten. Erfindungsgemäße Kieselsäuren können auch aus Fraktionen von gefällten und pyrogenen Kieselsäuren hergestellt werden.

Für Fällungskieselsäuren als Trägermaterial sind verschiedene Fällmethoden bekannt und können z. B. in EP 0 937 755 oder EP 0 643 015 nachgelesen werden. In den Beispielen sind exemplarisch zwei Fällungskieselsäuren aus verschiedenen Herstellverfahren zur erfindungsgemäßen inhomogenen Kieselsäure verarbeitet worden. Es ist auch möglich, hydrophobierte Kieselsäurefraktionen mit nicht behandelten Kieselsäurefraktionen zur erfindungsgemäßen Kieselsäure zu kombinieren.

Die Kieselsäurefraktionen können Fällungskieselsäuren oder pyrogene Kieselsäuren sein, wobei eine Mischung der Fraktionen an verschiedenen Prozeßschritten, die üblicherweise bei der Herstellung von Kieselsäuren durchgeführt werden, erfolgen kann.

Bei Verwendung von Fraktionen aus Fällungskieselsäuren kann die Mischung nach der Fällung von Silikat mit einer Säure (in der Regel Wasserglas, d. h. Natriumsilikat mit Schwefelsäure) durch Vermischen der Fällungssuspensionen oder der nach Filtration der Suspensionen erhaltenen Filterkuchen sowie verflüssigten (resuspendierten) Filterkuchen erfolgen. Es ist auch möglich, bereits hergestellte, getrocknete oder hydrophibierte Kieselsäurefraktionen als Feststoff den Suspensionen oder den Filterkuchen zuzusetzen.

Ein weiterer Gegenstand der Erfindung ist ein Verfahren zur Herstellung von Kieselsäuren, enthaltend mindestens zwei Kieselsäurefraktionen, wobei mindestens zwei Kieselsäurefraktionen, die sich in mindestens einem Meßwert für BET-Oberfläche, CTAB-Oberfläche und DBP-Aufnahme um mindestens 10 % unterscheiden, miteinander vermischt werden.

Der Anteil der jeweiligen Fraktionen in der Suspension bzw. an der Kieselsäure sollte jeweils zwischen 5 und 95 Gew.-%, bezogen auf die trockene Kieselsäure, betragen.

Die Kieselsäure wird bevorzugt in Partikelform mit einem mittleren Durchmesser von über 80, insbesondere über 100, besonders bevorzugt über 200 µm, z. B. durch die Sprühtrocknung erhalten. Die Sprühtrocknung der Suspension kann z. B. gemäß US 4 097 771 durchgeführt werden.

Die erfindungsgemäßen Kieselsäuren können daher als Trägermaterial insbesondere zur Adsorption von flüssigen Wirkstoffen verwendet werden.

Die erfindungsgemäßen Kieselsäuren sind insbesondere als Träger für Vitamine (A, B, C, E) ggf als Acetat, Proteine, Enzyme oder Cholinchlorid verwendbar.

Außerdem kann die Kieselsäure als Träger für katalytisch aktive Substanzen verwendet werden.

Weiterhin können die erfindungsgemäßen Kieselsäuren in allen Anwendungsgebieten verwendet werden, in denen üblicherweise Kieselsäuren eingesetzt werden, wie z. B. in Batterieseparatoren, Anti-Blocking-Mittel, Mattierungsmittel in Lacken, Papierstrichen oder Entschäumer.

Die erfindungsgemäße Kieselsäure oder die Kieselsäurefraktionen können mit Silanen, Silikonöl und/oder mit Organosilanen in bekannter Weise modifiziert, d. h. hydrophobiert werden.

Durchführung der Bestimmung des Alpine-Siebrückstandes:

Ein weiterer Gegenstand der Erfindung ist ein Verfahren zur Herstellung von Kieselsäuren, enthaltend mindestens zwei Kieselsäurefraktionen, wobei mindestens zwei Kieselsäurefraktionen, die sich in mindestens einem Meßwert für BET-Oberfläche, CTAB-Oberfläche und DBP-Aufnahme um mindestens 10 % unterscheiden, miteinander vermischt werden.

Der Anteil der jeweiligen Fraktionen in der Suspension bzw. an der Kieselsäure sollte jeweils zwischen 5 und 95 Gew.-%, bezogen auf die trockene Kieselsäure, betragen.

Die Kieselsäure wird bevorzugt in Partikelform mit einem mittleren Durchmesser von über 80, insbesondere über 100, besonders bevorzugt über 200 µm, z. B. durch die Sprühtrocknung erhalten. Die Sprühtrocknung der Suspension kann z. B. gemäß US 4 097 771 durchgeführt werden.

Die erfindungsgemäßen Kieselsäuren können daher als Trägermaterial insbesondere zur Adsorption von flüssigen Wirkstoffen verwendet werden.

Die erfindungsgemäßen Kieselsäuren sind insbesondere als Träger für Vitamine (A, B, C, E) ggf. als Acetat, Proteine, Enzyme oder Cholinchlorid verwendbar.

Außerdem kann die Kieselsäure als Träger für katalytisch aktive Substanzen verwendet werden.

Weiterhin können die erfindungsgemäßen Kieselsäuren in allen Anwendungsgebieten verwendet werden, in denen üblicherweise Kieselsäuren eingesetzt werden, wie z. B. in Batterieseparatoren, Anti-Blocking-Mittel, Mattierungsmittel in Lacken, Papierstrichen oder Entschäumer.

Die erfindungsgemäße Kieselsäure oder die Kieselsäurefraktionen können mit Silanen, Silikonöl und/oder mit Organosilanen in bekannter Weise modifiziert, d. h. hydrophobiert werden.

Durchführung der Bestimmung des Alpine-Siebrückstandes:

Zur Bestimmung des Siebrückstandes wird die Kieselsäure- oder Silikat-Probe durch ein 500 µm Sieb abgesiebt, um eventuell vorhandene Entlüftungsagglomerate zu zerstören. Dann werden 10 g der gesiebten Probe auf das Luftstrahlsieb gegeben, das mit einem 63 µm Siebgewebe bestückt ist und bei 200 mm Wassersäule-Unterdruck abgesiebt. Kieselsäure- oder Silikatpartikel, die sich am Siebdeckel des Gerätes absetzen, werden durch vorsichtiges Schlagen auf den Knopf des Siebdeckels abgeklopft. Der Siebvorgang dauert im allgemeinen 5 Minuten. Er ist beendet, wenn der Rückstand konstant bleibt, was meistens am rieselfähigen Aussehen zu erkennen ist. Zur Sicherheit siebt man dann noch eine weitere Minute.

Bei Agglomeraten, die sich eventuell bilden, wird der Siebvorgang kurz unterbrochen und die Agglomerate mit einem Pinsel unter leichtem Druck zerstört. Nach der Siebung wird der Siebrückstand vorsichtig vom Luftstrahlsieb geklopft und zurückgewogen. Der Siebrückstand wird in Prozenten angegeben, immer in Verbindung mit der Maschenweite des Siebes.

Berechnung:

$$\% \text{ Siebrückstand} = \frac{A \cdot 100}{E}$$

A = Auswaage in g
E = Einwaage in g

Geräte

Alpine Luftstrahlsieb, Labortyp S 200
Staubsauger oder Gebläse
Luftstrahlsieb mit Siebgewebe 63 um nach DIN 4188
Präzisionswaage

Die folgenden Beispiele sollen die Erfindung näher erläutern, ohne ihren Umfang zu beschränken.

Es wurden zwei Kieselsäurefraktionen A gemäß US 1 043 282 bzw. DE 24 47 013 und B gemäß DE 31 44 299 hergestellt und die aus den Fällungen erhaltenen Suspensionen in der im folgenden beschriebenen Weise weiter umgesetzt.

**Beispiel 1**

Die Fällsuspensionen der Kieselsäurefraktionen A und B wurden im Verhältnis 50:50 gemischt. Dazu wurde 80 kg der Fällungskieselsäure A (Feststoffgehalt ca. 46 g/l) mit 80 kg der Fällungskieselsäure B (Feststoffgehalt ca. 64 g/l) in einem gerührten Behälter gemischt. Die erhaltenen Mischung wurde filtriert, der Filterkuchen mit etwas Säure verflüssigt und auf einem Düsenturmtrockner versprüht. Die Analysendaten sind in Tabelle 1 zusammengefaßt.

**Beispiel 2**

Die Fällsuspensionen der Fällungskieselsäuren A und B wurden im Verhältnis 70:30 gemischt. Dazu wurde 112 kg der Fällungskieselsäure A (Feststoffgehalt ca. 46 g/l) mit 48 kg der Fällungskieselsäure B (Feststoffgehalt ca. 64 g/l) in einem gerührten Behälter gemischt. Die erhaltene Mischung wurde filtriert, der Filterkuchen mit etwas Säure verflüssigt und auf einem Düsenturmtrockner versprüht. Die Analysendaten sind in Tabelle 1 zusammengefaßt.

**Beispiel 3**

Die Fällsuspensionen der Fällungskieselsäuren A und B wurden im Verhältnis 30:70 gemischt. Dazu wurde 43,8 kg der Fällungskieselsäure A (Feststoffgehalt ca. 46 g/l) mit 102,2 kg der Fällungskieselsäure B (Feststoffgehalt ca. 64 g/l) in einem gerührten Behälter gemischt. Die erhaltene Mischung wurde filtriert, der Filterkuchen mit etwas Säure verflüssigt und auf einem Düsenturmtrockner versprüht. Die Analysendaten sind in Tabelle 1 zusammengefaßt.

**Beispiel 4**

Es wurde eine Mischung der getrockneten Kieselsäurefraktionen (50:50) hergestellt.

**Tabelle 1:**

Vergleich der Analysendaten aus den Beispielen 1-3 und der Kieselsäurefraktionen A und B:

| | | Kieselsäure-fraktion A | Kieselsäure-fraktion B | Unterschiede der Fraktionen A : B in % | Beispiel 1 | Beispiel 2 | Beispiel 3 | Beispiel 4 |
|---|---|---|---|---|---|---|---|---|
| Glühverlust DIN | % | 5,0 | 5,0 | | 3,7 | 2,7 | 3,4 | 10,1 |
| Wassergehalt | % | 5,0 | 4,5 | 10 | 5,3 | 5,3 | 5,5 | 5,6 |
| pH-Wert /A. Z. | | 6,5 | 6,0 | 7,6 | 6,2 | 6,4 | 6,4 | 6,6 |
| Leitfähigkeit | $\mu$S | 800 | 700 | 12,5 | 600 | 740 | 610 | 750 |
| BET-Oberfläche | $m^2/g$ | 195 | 195 | 00 | 354 | 403 | 284 | 302 |
| CTAB- Oberfläche | $m^2/g$ | 175 | 350 | 50 | 271 | 302 | 232 | 256 |
| DBP-Aufnahme | g/100g | 263 | 335 | 21,5 | 281 | 287 | 265 | 296 |
| Stampfdichte | g/l | 280 | 180 | 36,7 | 200 | 185 | 217 | 222 |
| Alpine Siebrückstand 63 $\mu$m | % | 80 | > = 20 | | 99 | 91 | 99 | 52 |
| Alpine Siebrückstand 180 $\mu$m | % | > = 4 | 1 | | 82 | 18 | 87 | 4,5 |
| Alpine Siebrückstand 250 $\mu$m | % | n. b. | n. b. | | 75 | 1,1 | 48 | |

EP 1 241 134 A2

**Anwendungstechnische Eigenschaften der erfindungsgemäßen Kieselsäure**

**Fließeigenschaften der Kieselsäure**

Die erfindungsgemäß hergestellten Produkte gemäß den Beispielen 1-3 weisen eine sehr gute Eigenfließfähigkeit auf.

| | Methode | Einheit | Beschreibung der Methode | KS gemäß Bsp. 1 | KS gemäß Bsp. 2 | KS gemäß Bsp. 3 |
|---|---|---|---|---|---|---|
| Fließeigenschaft | Glasauslaufgefäß | (Note) | | 1 | 1 | 1 |
| | Schüttkegelhöhe | [mm] | | 9 | 13 | 8 |

**Maximale Cholinchloridaufnahme**

Die maximale Cholinchloridaufnahme liefert eine wichtige Information über die Saugkapazität einer Kieselsäure. Da höher konzentrierte Adsorbate von Vorteil sind, ist eine möglichst hohe Saugkapazität gewünscht. Die maximale Cholinchloridaufnahme der inhomogenen Kieselsäuren ist deutlich höher als bei Produkten nach dem Stand der Technik.

| Methode | Einheit | KS gemäß Bsp. 1 | KS gemäß Bsp. 2 | KS gemäß Bsp. 3 | KS gemäß Bsp. 4 | Vergleichs beispiel Sipernat 2200 | Vergleichsbeispiel Sipernat 22 | Vergleichs beispiel Hubersil 5170 |
|---|---|---|---|---|---|---|---|---|
| Maximale Cholinchloridaufnahme | [g/100 g] | 300 | 295 | 268 | 280 | 245 | 240 | 165 |

**Fließfähigkeit eines Cholinchlorid Adsorbates**

Außer einer hohen Aufnahmekapazität für Flüssigkeiten ist gefordert, daß die resultierenden Adsorbate auch gut fließfähig sind. Als Beispiel wurde ein 50%iges Adsorbat von Cholinchlorid auf der entsprechenden Kieselsäure aus 66,6 g einer 75%igen wässrigen Cholinchloridlösung und 33,3 g der jeweiligen Kieselsäure hergestellt und die Fließfähigkeit mittels Glasauslaufgefäßen und Schüttkegelhöhe beurteilt. Die inhomogenen Kieselsäuren DTT 3120 und DTT 3140 liefern hierbei Vorteile gegenüber Standardkieselsäuren (Hubersil 5170).

| | Methode | Einheit | KS gemäß Bsp. 1 | KS gemäß Bsp. 2 | KS gemäß Bsp. 3 | Vergl.-Bsp. Hubersil 5170 |
|---|---|---|---|---|---|---|
| Fließeigenschaft 50% Cholinchlorid Adsorbat | Glasauslaufgefäß | (Note) | 2 | 5 | 1 | 6 |
| | Schüttkegelhöhe | [mm] | 18 | 32 | 24 | > 50 |

**Agglomeratanteil**

Der Agglomeratanteil gibt eine wesentliche Aussage darüber, ob eine Kieselsäure als Trägersubstanz geeignet ist: Ein hoher Agglomeratanteil ist unerwünscht, da er zu einem schlecht verarbeitbaren Adsorbat führt. Der Agglomeratanteil eines 50%igen Cholinchlorid-Konzentrates, hergestellt aus je 100 g der entsprechenden Kieselsäure und 200 g einer 75%igen wässrigen Cholinchloridlösung, ist bei den untersuchten inhomogenen Kieselsäuren mit 0,3 — 2,1 % sehr gering. Die Vergleichskieselsäuren liefern deutlich höhere Agglomeratanteile.

| | Meth ode | Ein heit | KS gemäß Bsp. 1 | KS gemäß Bsp. 2 | KS gemäß Bsp. 3 | Vergleichsbeispiel Sipernat 2200 | Vergleichsbeispiel Sipernat 22 | Vergleichsbeispiel HiSil SC72 |
|---|---|---|---|---|---|---|---|---|
| 50% Adsorbat von Cholinchl orid auf KS | Agglo mer atante il | [%] | 1,3 | 2,1 | 0,3 | 3,7 | 2,8 | 2,7 |

**Sauggeschwindigkeit**

Für die Anwendung ist weiterhin die Sauggeschwindigkeit wichtig, da bei der Herstellung von Adsorbaten in der Technik hohe Durchsätze und damit kurze Verweilzeiten im Mischer angestrebt werden. Bei den untersuchten inhomogenen Kieselsäuren ist die Sauggeschwindigkeit für Vitamin E-acetat besser als die der Vergleichprodukte Sipernat 2200 und Hubersil 5170.

| Methode | Einheit | KS gemäß Bsp. 1 | KS gemäß Bsp. 2 | KS gemäß Bsp. 3 | KS gemäß Bsp. 4 | Vergleichsbeispiel Sipernat 2200 | Vergleichsbeispiel Hubersil 5170 |
|---|---|---|---|---|---|---|---|
| Sauggeschwindigkeit Vitamin E-Acetat | (Note) | 2,5 | 2,5 | 3,0 | 2,0 | 4,5 | 5 |

Die Meßmethoden der Fließeigenschaften, Cholinchloridaufnahme, Agglomeratanteil und Sauggeschwindigkeit werden gemäß der Firmenschrift "Synthetische Pigmente als Fließhilfsmittel und als Trägersubstanz", Schriftenreihe Pigmente Nr. 31, Degussa AG 1992, ebenso Nr. 1 und Nr. 30.

Die Untersuchungsergebnisse belegen, daß die neuen inhomogenen Trägerkieselsäuren zur Herstellung von hochkonzentrierten Adsorbaten geeignet, gut fließfähig und wenig staubend sind. Gezeigt wurde dies am Beispiel der Absorption von Vitamin E-acetat und 75%iger wässriger Cholinchloridlösung. Beide Produkte werden als Adsorbat in der Futtermittelindustrie verwendet. In der Praxis ist auch die Herstellung von anderen hochkonzentrierten Adsorbaten, wie Melaminharzen (Additiv in der Gummiindustrie), Säuren z.B. Ameisen- oder Phosphorsäure (Futtermittelindustrie), Pigmente z.B. Tagetesextrakte (Futtermittelindustrie) denkbar.

**Patentansprüche**

1. Kieselsäure, enthaltend mindestens zwei Kieselsäurefraktionen,
   **dadurch gekennzeichnet,**
   **dass** die mindestens zwei Kieselsäurefraktionen sich in mindestens einem Meßwert für BET-Oberfläche, CTAB-Oberfläche und DBP-Aufnahme um mindestens 10 % unterscheiden.

2. Kieselsäure nach Anspruch 1,
   **dadurch gekennzeichnet,**
   **dass** sie in Form von Partikeln mit einem mittleren Teilchendurchmesser von über 80 $\mu$m vorliegt.

3. Kieselsäure nach einem der Ansprüche 1 oder 2,
   **dadurch gekennzeichnet,**
   **dass** die Kieselsäure die folgenden physikalisch-chemischen Daten aufweist:

   | BET-Oberfläche | 100 — 900 m$^2$/g |
   |---|---|
   | CTAB-Oberfläche | 100 — 500 m$^2$/g |
   | DBP-Aufnahme | 150 — 350 g/100 g. |

4. Kieselsäure nach einem der Ansprüche 1 bis 3,
   **dadurch gekennzeichnet,**
   **dass** der jeweilige Anteil einer Kieselsäurefraktion in der Kieselsäure zwischen 5 und 95 Gew.-% liegt.

5. Kieselsäure nach einem der Ansprüche 1 bis 4,
   **dadurch gekennzeichnet,**
   **dass** die Kieselsäure hydrophobiert wird.

6. Kieselsäure nach einem der Ansprüche 1 bis 5,
   **dadurch gekennzeichnet,**
   **dass** mindestens eine Kieselsäurefraktion hydrophobiert wird.

7. Kieselsäure nach einem der Ansprüche 1 bis 6,
   **dadurch gekennzeichnet,**
   **dass** eine oder mehrere Kieselsäurefraktionen aus einer Fällungskieselsäure bestehen.

8. Kieselsäure nach einem der Ansprüche 1 bis 7,
   **dadurch gekennzeichnet,**
   **dass** die Kieselsäurefraktionen durch Fällung von Silikat mit einer Säure hergestellt und die so erhaltenen Fällungssuspensionen gemischt werden.

9. Kieselsäure nach einem der Ansprüche 1 bis 7,
   **dadurch gekennzeichnet,**
   **dass** die Kieselsäurefraktionen durch Fällung von Silikat mit einer Säure hergestellt, die Fällungssuspension abfiltriert und die so erhaltenen Filterkuchen gemischt werden.

10. Kieselsäure nach einem der Ansprüche 1 bis 7,

**dadurch gekennzeichnet,**
**dass** die Kieselsäurefraktionen durch Fällung von Silikat mit einer Säure hergestellt, die Filterkuchen oder bereits getrocknete Kieselsäure verflüssigt und die so erhaltenen Suspensionen gemischt werden.

**11.** Kieselsäure nach einem der Ansprüche 1 bis 6,
**dadurch gekennzeichnet,**
**dass** eine oder mehrere Kieselsäurefraktionen aus einer pyrogenen Kieselsäure bestehen.

**12.** Kieselsäure nach einem der Ansprüche 1 bis 7 und 11,
**dadurch gekennzeichnet,**
**dass** die Kieselsäurefraktionen im trockenen Zustand gemischt werden.

**13.** Verfahren zur Herstellung von Kieselsäuren, enthaltend mindestens zwei Kieselsäurefraktionen,
**dadurch gekennzeichnet,**
**dass** mindestens zwei Kieselsäurefraktionen, die sich in mindestens einem Meßwert für BET-Oberfläche, CTAB-Oberfläche und DBP-Aufnahme um mindestens 10 % unterscheiden, miteinander vermischt werden.

**14.** Verfahren nach Anspruch 13,
**dadurch gekennzeichnet,**
**dass** die Kieselsäure in Form von Partikeln mit einem mittleren Teilchendurchmesser von über 80 μm vorliegt.

**15.** Verfahren nach einem der Ansprüche 13 oder 14,
**dadurch gekennzeichnet,**
**dass** die Kieselsäure die folgenden physikalisch-chemischen Daten aufweist:

| BET-Oberfläche | 100 — 900 m$^2$/g |
|---|---|
| CTAB-Oberfläche | 100 — 500 m$^2$/g |
| DBP-Aufnahme | 150 — 350 g/100 g. |

**16.** Verfahren nach einem der Ansprüche 13 bis 15,
**dadurch gekennzeichnet,**
**dass** der jeweilige Anteil einer Kieselsäurefraktion in der Kieselsäure zwischen 5 und 95 Gew.-% liegt.

**17.** Verfahren nach einem der Ansprüche 13 bis 16,
**dadurch gekennzeichnet,**
**dass** die Kieselsäure hydrophobiert wird.

**18.** Verfahren nach einem der Ansprüche 13 bis 16,
**dadurch gekennzeichnet,**
**dass** mindestens eine Kieselsäurefraktion hydrophobiert wird.

**19.** Verfahren nach einem der Ansprüche 13 bis 18,
**dadurch gekennzeichnet,**
**dass** eine oder mehrere Kieselsäurefraktionen aus einer Fällungskieselsäure bestehen.

**20.** Verfahren nach einem der Ansprüche 13 bis 19,
**dadurch gekennzeichnet,**
**dass** die Kieselsäurefraktionen durch Fällung von Silikat mit einer Säure hergestellt und die so erhaltenen Fällungssuspensionen gemischt werden.

**21.** Verfahren nach einem der Ansprüche 13 bis 19,
**dadurch gekennzeichnet,**
**dass** die Kieselsäurefraktionen durch Fällung von Silikat mit einer Säure hergestellt, die Fällungssuspension abfiltriert und die so erhaltenen Filterkuchen gemischt werden.

**22.** Verfahren nach einem der Ansprüche 13 bis 19,
**dadurch gekennzeichnet,**

**dass** die Kieselsäurefraktionen durch Fällung von Silikat mit einer Säure hergestellt, die Filterkuchen oder bereits getrocknete Kieselsäure verflüssigt und die so erhaltenen Suspensionen gemischt werden.

23. Verfahren nach einem der Ansprüche 13 bis 18,
    **dadurch gekennzeichnet,**
    **dass** eine oder mehrere Kieselsäurefraktionen aus einer pyrogenen Kieselsäure bestehen.

24. Verfahren nach einem der Ansprüche 13 bis 19 und 23,
    **dadurch gekennzeichnet,**
    **dass** die Kieselsäurefraktionen im trockenen Zustand gemischt werden.

25. Verwendung der Kieselsäure gemäß einem der Ansprüche 1 bis 12 als Trägermaterial

26. Verwendung der Kieselsäure nach einem der Ansprüche 1 bis 12 als Trägermaterial für Vitamine, Vitamin-Acetate, Cholinchlorid, Proteine oder Enzyme.

27. Verwendung der Kieselsäure nach Anspruch 26 als Trägermaterial für katalytisch aktive Substanzen.